(19) Europäisches Patentamt
European Patent Office
Office européen des brevets

(11) Veröffentlichungsnummer: **0 027 961**
**B1**

# (12) EUROPÄISCHE PATENTSCHRIFT

(45) Veröffentlichungstag der Patentschrift: **12.01.83**

(51) Int. Cl.³: **C 07 J 1/00,** A 61 K 31/565

(21) Anmeldenummer: **80106289.4**

(22) Anmeldetag: **16.10.80**

## (54) 17-alpha-Substituierte Steroide, diese enthaltende Präparate sowie Verfahren zu ihrer Herstellung.

(30) Priorität: **26.10.79 DE 2943776**

(43) Veröffentlichungstag der Anmeldung: **06.05.81 Patentblatt 81/18**

(45) Bekanntmachung des Hinweises auf die Patenterteilung: **12.01.83 Patentblatt 83/2**

(84) Benannte Vertragsstaaten: **AT BE CH DE FR GB IT LI LU NL SE**

(56) Entgegenhaltungen:
**FR-A-1 382 468**
**FR-A-2 319 367**

**COLLECTION OF CZECHOSLOVAK CHEMICAL COMMUNICATIONS, Band 29, Nr. 12, 1964, Seiten 3089–3095, Prague, CS, B. PELC: "Steroid derivatives. XXVIII. Preparation of 1-methyl-5 alpha-androstane derivatives"**

**COLLECTION OF CZECHOSLOVAK CHEMICAL COMMUNICATIONS, Band 30, Nr. 10, 1965, Seiten 3468–3472, Prague, CS, B. PELC: "Steroid derivatives. XXIX. 1-alpha-methylated 5-alpha-androstane derivatives substituted at C(3) and C(17)"**

**CHEMICAL ABSTRACTS, Band 65, Nr. 7, 26. September 1966, Zusammenfassung Nr. 10641h, Seite 10641–10642, Columbus, Ohio, US**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Wiechert, Rudolf, Dr. Prof., Petzower Strasse 8 A, D-1000 Berlin 39 (DE)**
Erfinder: **Bittler, Dieter, Bölkauer Pfad 11, D-1000 Berlin 27 (DE)**
Erfinder: **Schleusener, Annerose, Dr., Ilsensteinweg 1 b, D-1000 Berlin 38 (DE)**
Erfinder: **Albring, Manfred, Dr., Am Dachsbau 34 a, D-1000 Berlin 27 (DE)**

**EP 0 027 961 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 17-alpha-Substituierte Steroide, diese enthaltende Präparate sowie Verfahren zu ihrer Herstellung

Die Erfindung betrifft den in den Patentansprüchen gekennzeichneten Gegenstand.

Die Verbindung der allgemeinen Formel I gemäss Anspruch 1 mit $R_1$ und $R_2$ in der Bedeutung von Wasserstoff und X in der Bedeutung von Sauerstoff mit der Bezeichnung «Mesterolon» ist ein starkes oral wirksames Androgen. Mesterolon (17-alpha-Methyl-androstan-17β-ol-3-on) wird zum Beispiel beschrieben in der deutschen Patentschrift 1152100 in Arzneimittel-Forsch. 16,4: 455-466 (1966), in der japanischen Patentschrift 8347 (66) ref. in C.A. 65, 1966, 10641, in Collect. of Czecheslow. Chem. Comm. 29, 1964, 3089ff und 30, 1965, 3468ff. und in der französischen Patentschrift 1382468.

Aus FR-A-2319367 sind 17α-R-Androst-4-en-17β-ol-3-one, wo R $C_3H_7$ oder $C_4H_9$ bedeutet, bekannt. Bei der topischen Anwendung zeigen sie antiandrogene Eigenschaften.

Es wurde nun gefunden, dass die vom Mesterolon abgeleiteten 17alpha-substituierten Steroide der allgemeinen Formel I bei der topischen Anwendung antiandrogene Eigenschaften zeigen. Die 17alpha-substituierten Steroide der allgemeinen Formel I wirken einem vorhandenen oder zugeführten Androgen entgegen. So wird mit den Verbindungen der allgemeinen Formel I das durch Testosteronpropionat stimulierte Wachstum der Flankenorgane und der Talgdrüsen der Ohren bei kastrierten männlichen Hamstern gehemmt, während andere androgen-abhängige Organe wie Prostata und Samenblasen von den Verbindungen der allgemeinen Formel I nicht signifikant beeinflusst werden.

Die topische antiandrogene Wirkung wurde wie folgt bestimmt:

Männliche, fertile Hamster im Gewicht von etwa 80 g werden kastriert und mit täglich 0,1 mg Testosteronpropionat subcutan substituiert. Das rechte Ohr und das rechte Flankenorgan werden täglich zweimal mit 0,01 ml einer 3%igen Lösung des zu testenden Antiandrogens in einem organischen Lösungsmittel, vorzugsweise Aceton, 3 Wochen lang behandelt. Am 22. Tag werden die Tiere mit Ether getötet, Prostata, Samenblasen und Flankenorgane präpariert und gewogen, die Ohren histologisch weiterverarbeitet und die Areale der Talgdrüsen gemessen. Bei den Ohren weiterer behandelter Tiere wird der Einbau von $^{14}C$-markierter Glukose in die Fette der Talgdrüsen gemessen.

Es hat sich gezeigt, dass die Talgdrüsenkonglomerate als Flankenorgane und die gut abgrenzbaren und planimetrisch gut erfassbaren Talgdrüsen an den ventralen Seiten der Ohrmuscheln des Hamsters in ihrer Grösse androgenabhängig sind. Als Parameter für die Talgdrüsenfunktion wird der Einbau $^{14}C$-markierter Vorstufen der Lipidsynthese gemessen.

Durch Vergleich der Areale der Talgdrüsen, der Gewichte der Flankenorgane und der Lipogenese der jeweils mit dem Antiandrogen behandelten Seite mit der Lösungsmittelkontrolle erhält man ein Mass für den lokalen Effekt des Antiandrogens.

In einem Versuch wurden 21 Tage lang 2mal täglich 0,01 ml einer 3%igen äthanolischen Lösung von 17β-Hydroxy-1α-methyl-17α-n-propyl-5α-androstan-3-on (A) auf das rechte Flankenorgan und das rechte Ohr von kastrierten syrischen Goldhamstern aufgetragen. Die Goldhamster wurden ausserdem täglich mit 0,1 mg Testosteronpropionat in Benzylbenzoat/Rizinusöl im Verhältnis 1:100 subcutan behandelt. Am 22. Tag wurden die Tiere getötet, Samenblasen, Prostata und Flankenorgane präpariert und gewogen und die Areale der Talgdrüsen der histologisch weiterverarbeiteten Ohren gemessen. Pro Versuch wurden 10 Tiere eingesetzt.

Die Ergebnisse gehen aus der folgenden Tabelle hervor:

| | Samenblasen | Prostata | Flankenorgane | | Talgdrüsen-Areale | |
|---|---|---|---|---|---|---|
| | | | rechts | links | rechts | links |
| | mg | mg | mg | mg | mm² | mm² |
| A | 1101 ± 34,58 | 355 ± 21,91 | 35 ± 1,92 | 67 ± 3,64 | 0,0634 ± 0,0054 | 0,1516 ± 0,021 |
| Kontrolle | 1106 ± 46,52 | 404 ± 23,82 | 70 ± 3,53 | 72 ± 3,67 | 0,2309 ± 0,026 | 0,2428 ± 0,023 |

Es zeigt sich eine deutliche Reduktion des Gewichts des behandelten rechten Flankenorgans und eine Reduktion der Talgdrüsenfläche (Areals) des behandelten rechten Ohrs. Bei der lokalen Anwendung von A wird das Samenblasengewicht fast gar nicht und das Prostatagewicht nur geringfügig beeinflusst.

In einem weiteren Versuch wurde die Sebumproduktion der Talgdrüsen von wie oben behandelten Hamsterohren durch Einbau von ($^{14}C$) Natriumacetat in die Lipide der Talgdrüsenzellen in vitro und anschliessende Bestimmung der Radioaktivität im Lipidextrakt gemessen. Aus der Radioaktivität der Einzelproben wurden die Mittelwerte und die Standardabweichungen berechnet. Die prozentuale Hemmung der Lipogenese der behandelten rechten Ohren wurden im Vergleich zur Kontrollgruppe, das heisst den mit dem Lösungsmittel behandelten rechten Ohren

berechnet. Es zeigt sich bei diesem Versuch eine deutliche und dosisabhängige Reduktion der Lipogenese an den behandelten Ohren.

| A | Reduktion der Lipogenese |
|---|---|
| 3% | 43,8 ± 7,0% |
| 1% | 51,9 ± 7,6% |
| 0,3% | 26,25 ± 21,0% |
| 0,1% | 33,6 ± 14,0% |

Zur topischen Anwendung können die 17α-Alkylsteroide der allgemeinen Formel I mit üblichen Trägersubstanzen zu Lösungen, Suspensionen, Gels, Salben, Cremes, Puder oder anderen Präparaten verarbeitet werden. Geeignete Trägersubstanzen sind zum Beispiel Wasser, Ethanol, Propanol, Glycerin, Methylcellulose, Hydroxypropylcellulose, Carboxypolymethylen usw. Das Antiandrogen liegt vorzugsweise in einer Konzentration von 0,05 bis 5,0 Gew.-% bezogen auf das Gesamtgewicht des Präparats vor. Die Präparate können zur topischen Behandlung von Erkrankungen, wie Akne, Seborrhoe, Alopezie und Hirsutismus, verwendet werden.

Die Steroide der allgemeinen Formel I gemäss Anspruch 1 enthalten in 17α-Stellung einen aliphatischen Kohlenwasserstoffrest $R_2$. Unter einem aliphatischen Kohlenwasserstoffrest $R_2$ soll eine 3-6 Kohlenstoffatome enthaltende - vorzugsweise geradkettige - Alkyl- oder Alkenylgruppe verstanden werden. Geradkettige Alkylgruppen $R_2$ sind beispielsweise die Propyl-, Butyl-, Pentyl- und Hexylgruppe. Verzweigtkettige Alkylgruppen sind beispielsweise die Isopropyl-, Isobutyl- und Isoamylgruppe. Geeignete Alkenylgruppen sind beispielsweise die Propenyl- und Isobutenylgruppe.

In 17β- und gegebenenfalls auch in 3β- oder 3α-Stellung enthalten die Steroide der allgemeinen Formel I eine freie oder veresterte oder veretherte Hydroxygruppe ($OR_1$ bzw. $OR_3$).

Die Ester $OR_1$ oder $OR_3$ leiten sich von den in der Steroidchemie üblichen Säuren ab. Beispielsweise genannt seien organische Carbon- und Sulfonsäuren mit 1-17 Kohlenstoffatomen, vorzugsweise verwendet werden organische Carbonsäuren mit 1-7 Kohlenstoffatomen. Beispiele für die Acylgruppe $R_1$ und $R_3$ sind Formyl, Acetyl, Propionyl, Butyryl, Isobutyryl, Caproyl, Heptanoyl, Chloracetyl, Trifluoracetyl, Glykoloyl, Succinyl, Glutaryl, Adipoyl, Dimethylaminopropionyl, Benzoyl, Nicotinoyl, Isonicotinoyl usw.

Die Ether $OR_1$ und $OR_3$ enthalten eine Alkyl- die Cyclopentyl- oder Tetrahydropyranylgruppe. Alkylgruppen $R_1$ und $R_3$ sollen vorzugsweise 1-5 Kohlenstoffatome enthalten und gegebenenfalls durch ein Sauerstoffatom unterbrochen sein. Beispiele für die gegebenenfalls durch ein Sauerstoffatom unterbrochene Alkylgruppe $R_1$ und $R_3$ sind Methyl, Ethyl, Methoxymethyl, Methoxyethyl, Ethoxyethyl, Propyl, Butyl, Pentyl usw.

Das in Anspruch 14 gekennzeichnete Verfahren wird in an sich bekannter Weise durchgeführt. Für die Umsetzung der 17-Ketogruppe mit einer den Rest $R_2$ abgebenden metallorganischen Verbindung muss die Ketogruppe in 3-Stellung geschützt sein. Die Schutzgruppe Y in Verbindungen der allgemeinen Formel II soll durch saure Hydrolyse abspaltbar sein. Nach einer bevorzugten Ausführungsform ist die Ketogruppe in 3-Stellung durch Ketalbildung geschützt. Die Ketalreste Y leiten sich von den üblicherweise zum Schutz freier Oxogruppen vewendeten Alkoholen und Thioalkoholen ab, beispielsweise genannt seien: Ethylenglykol, 2,2-Dimethylpropandiol-(1,3) und Ethandithiol-(1,2). Die 3-Ketogruppe kann aber auch durch Enolether-, Enolester- oder Enaminbildung geschützt sein.

Die Umsetzung der 17-Ketoverbindung der allgemeinen Formel II erfolgt nach bekannten Methoden mit einer metallorganischen Verbindung ($R_2$-Metall), insbesondere mit $R_2$-Lithium, wie zum Beispiel n-Butyllithium. Die metallorganische Verbindung kann auch in der Reaktionslösung aus dem Halogenalkan und dem Alkalimetall, wie zum Beispiel 1-Brompentan oder 1-Bromhexan und Lithium, hergestellt werden. Die Reaktion wird in einem inerten Lösungsmittel, wie zum Beispiel Ether, Tetrahydrofuran, Hexan usw. durchgeführt. Die Reaktionstemperatur liegt zwischen 0 und 50°C, vorzugsweise bei Raumtemperatur.

Zur Herstellung der 17α-Propylverbindung ($R_2$ = Propyl) wird zweckmässigerweise zunächst durch Grignardierung mit Allylmagnesiumbromid die 17α-Allylverbindung gebildet und diese anschliessend zur 17α-Propylverbindung hydriert. Die Hydrierung erfolgt mit katalytisch angeregtem Wasserstoff. Als Katalysatoren kommen zum Beispiel Palladium auf Kohle in Methanol oder Tristriphenylphosphinrhodiumchlorid in Aceton in Frage. Analog wird die 17α-Isobutyl- über die 17α-Isobutenylverbindung hergestellt.

Die Abspaltung der 3-Ketoschutzgruppe (Y), die vor oder gegebenenfalls auch nach der möglichen Veresterung oder Veretherung der 17-Hydroxygruppe erfolgen kann, wird nach den dem Fachmann bekannten Methoden durch saure Hydrolyse durchgeführt. Zur Schutzgruppenabspaltung kommen Mineralsäuren, wie zum Beispiel Perchlorsäure, Schwefelsäure, Salzsäure, oder organische Säuren, wie zum Beispiel Oxalsäure, in Betracht. Die Spaltung wird vorzugsweise in alkoholischer Lösung oder in anderen polaren Lösungsmitteln, wie zum Beispiel Aceton, bei Temperaturen zwischen etwa 20 und 100°C durchgeführt.

Für die sich gegebenenfalls anschliessende Veresterung der tertiären 17-Hydroxygruppe kommen die üblicherweise in der Steroidchemie zur Veresterung tertiärer Steroidalkohole angewendeten Verfahren in Frage. Beispielsweise genannt seien die Umsetzung mit Säuren oder Säureanhydriden in Gegenwart starker Säuren, wie zum Beispiel Trifluoressigsäure oder p-Toluolsulfonsäure, bei Temperaturen zwischen etwa 10 und 50°C, oder die Umsetzung mit einem Säureanhydrid in Gegenwart eines tertiären Amins, wie

zum Beispiel Pyridin oder Collidin, bei etwa 20 bis 200°C. Werden Pyridin und 4-(Dimethylamino)-pyridin als tertiäre Amine gemeinsam eingesetzt, kann die Veresterung der tertiären 17-Hydroxygruppe auch bei Raumtemperatur durchgeführt werden.

Zur Veretherung der 17-Hydroxygruppe bzw. der Hydroxygruppen in 3- oder in 3- und 17-Stellung dienen alkylierende Verbindungen, wie zum Beispiel Alkylhalogenide. Die Veretherung erfolgt in an sich bekannter Weise in Gegenwart einer starken Base, wie Natronlauge, unter Verwendung eines polaren Lösungsmittels, wie Hexamethylphosphorsäuretriamid, bei 0-50°C oder in Gegenwart einer starken Base, wie Natriumhydrid, unter Verwendung eines Ethers, wie Tetrahydrofuran, bei 30-100°C.

Zur Herstellung von Alkylethern, deren Kohlenstoffkette durch ein Sauerstoffatom unterbrochen und gegebenenfalls ringgeschlossen ist, werden die Hydroxyverbindungen mit Dihydropyran oder Alkylvinylethern in Gegenwart einer starken Säure, wie p-Toluolsulfonsäure oder Phosphoroxychlorid, in die entsprechenden Tetrahydrophyranyl- oder Alkoxyethylether überführt. Die Reaktion wird vorzugsweise in Gegenwart von inerten Lösungsmitteln, wie Chloroform, Dichlormethan, Tetrahydrofuran, Dioxan etc., bei einer Reaktionstemperatur von -20°C bis 100°C durchgeführt. Zur Herstellung von Methoxymethylethern wird die Hydroxyverbindung zum Beispiel mit Formaldehyddimethylacetal in wasserfreiem Dichlormethan in Gegenwart von Phosphorpentoxid bei Raumtemperatur umgesetzt.

Die Reduktion der Ketogruppe in 3-Stellung kann nach an sich bekannten Methoden durch Hydrieren mit einem Metallhydrid erfolgen. Als Wasserstoffdonatoren haben sich insbesondere komplexe Hydride, wie zum Beispiel Natriumborhydrid und Lithium-tri-(tert.-butoxy)-aluminiumhydrid bewährt. Die Reduktion mit Natriumborhydrid wird vorzugsweise in wässrig-alkoholischer Lösung und die mit Lithium-tri-(tert.-butoxy)-aluminiumhydrid in etherischer Lösung vorgenommen. Die Reduktion wird unter milden Bedingungen, vorzugsweise bei Temperaturen zwischen etwa 0 und 50°C, durchgeführt.

Für die anschliessende Veresterung der Hydroxygruppe in 3-Stellung sei beispielsweise die Umsetzung mit einem Säureanhydrid oder -halogenid in Gegenwart eines tertiären Amins, wie zum Beispiel Pyridin, Collidin oder Triethylamin, bei Raumtemperatur genannt. Die 3-Hydroxygruppe kann auch mit dem Säureanhydrid unter Verwendung einer starken Säure wie p-Toluolsulfonsäure oder mit der entsprechenden Säure und Trifluoressigsäureanhydrid bei Raumtemperatur verestert werden.

Bei der Veresterung in Gegenwart eines sauren Katalysators bei Raumtemperatur oder in Gegenwart eines basischen Katalysators bei erhöhter Temperatur von 20-200°C können auch die Hydroxygruppen in 3- und 17-Stellung gleichzeitig verestert werden.

Werden Pyridin oder Triethylamin und zusätzlich 4-(Dimethylamino)-pyridin als basische Katalysatoren angewandt, können beide Hydroxygruppen auch im basischen Milieu bei Raumtemperatur verestert werden.

Die als Ausgangsmaterialien verwendeten 17-Ketosteroide der allgemeinen Formel II werden nach an sich bekannten Methoden aus 1α-Methyl-5α-androstan-17α-ol-3-on durch Einführung einer Schutzgruppe in 3-Stellung und Oxydation in 17-Stellung hergestellt. Die Herstellung der Verbindungen der allgemeinen Formel II sei am Beispiel der Herstellung von 3,3-Ethylendioxy-1α-methyl-5α-androstan-17-on näher erläutert:

20 g 17β-Hydroxy-1α-methyl-5α-androstan-3-on werden in 500 ml Benzol mit 60 ml Ethylenglykol und 600 mg p-Toluolsulfonsäure 5,5 Stunden am Rückfluss mit Wasserabscheider gerührt. Nach dem Abkühlen wird die Reaktionslösung mit Ether verdünnt, mit Natriumhydrogencarbonatlösung und Wasser gewaschen und getrocknet. Es werden nach dem Eindampfen 23 g rohes 3,3-Ethylendioxy-1α-methyl-5α-androstan-17β-ol erhalten.

23 g 3,3-Ethylendioxy-1α-methyl-5α-androstan-17β-ol werden in 230 ml Dichlormethan mit 20 g Pyridiniumchlorochromat in Gegenwart von 20 g Natriumacetat 1 Stunde bei Raumtemperatur gerührt. Anschliessend wird die Reaktionslösung mit Ether verdünnt, von den unlöslichen Anteilen abfiltriert und das Filtrat mit Wasser gewaschen. Nach dem Trocknen und Eindampfen werden 21,5 g 3,3-Ethylendioxy-1α-methyl-5α-androstan-17-on als Rohprodukt erhalten.

Beispiel 1

800 mg Magnesiumspäne werden in 20 ml absolutem Ether mit 2 ml Allylbromid in 5 ml absolutem Ether zu Allylmagnesiumbromid umgesetzt. Zu dieser Lösung werden bei Raumtemperatur 2 g 3,3-Ethylendioxy-1α-methyl-5α-androstan-17-on in 5 ml Dichlormethan gegeben und 3 Stunden gerührt. Die eisgekühlte Reaktionslösung wird dann langsam mit gesättigter Ammoniumchloridlösung versetzt, mit Ether verdünnt, mit gesättigter Ammoniumchloridlösung und mit Wasser gewaschen. Nach dem Trocknen und Eindampfen werden 2,1 g 3,3-Ethylendioxy-1α-methyl-17α-(2-propenyl)-5α-androstan-17β-ol als Rohprodukt erhalten.

2,1 g 3,3-Ethylendioxy-1α-methyl-17α-(2-propenyl)-5α-androstan-17β-ol werden in 105 ml Methanol mit 210 mg Palladium auf Kohle (10%ig) bis zur Aufnahme von einem Äquivalent Wasserstoff hydriert. Der Katalysator wird abfiltriert und das Filtrat im Vakuum eingedampft. Es werden 2,1 g 3,3-Ethylendioxy-1α-methyl-17α-n-propyl-5α-androstan-17β-ol als Rohprodukt erhalten. Eine aus Diisopropylether umkristallisierte Probe schmilzt bei 150-150,5°C.

1,5 g 3,3-Ethylendioxy-1α-methyl-17α-n-propyl-5α-androstan-17β-ol werden in 30 ml Methanol und 3 ml Wasser mit 1,5 g Oxalsäure 2 Stunden bei Raumtemperatur gerührt. Es wird dann

mit Ether verdünnt, mit Wasser gewaschen und getrocknet. Nach dem Eindampfen wird der Rückstand an Silikagel chromatographiert, und es werden 1,1 g 17β-Hydroxy-1α-methyl-17α-n-propyl-5α-androstan-3-on als Öl erhalten. $[\alpha]_D^{23}$ + 7,5° (Chloroform).

Beispiel 2

1,25 g 3,3-Ethylendioxy-1α-methyl-5α-androstan-17-on werden in 12,5 ml absolutem Tetrahydrofuran bei Eiskühlung und Überleiten von Argon mit 3,5 ml Butyl-Lithiumlösung (15%ig in Hexan) versetzt und 22 Stunden bei Raumtemperatur gerührt. Es wird dann mit Wasser das überschüssige Reagenz zersetzt, die Reaktionslösung mit Ether verdünnt und mit Wasser gewaschen. Nach dem Trocknen und Eindampfen wird der Rückstand an Silikagel chromatographiert, und es werden 950 mg 17α-n-Butyl-3,3-ethylen-dioxy-1α-methyl-5α-androstan-17β-ol als Rohprodukt erhalten.

950 mg 17α-n-Butyl-3,3-ethylendioxy-1α-methyl-5α-androstan-17β-ol werden in 10 ml Methanol mit 1 ml 8 Vol.%iger Schwefelsäure 30 Minuten bei Raumtemperatur gerührt. Es wird mit Ether verdünnt, mit Wasser gewaschen und getrocknet. Der nach dem Eindampfen erhaltene Rückstand wird an Silikagel chromatographiert, und es werden 620 mg 17α-n-Butyl-17β-hydroxy-1α-methyl-5α-androstan-3-on als Öl erhalten. $[\alpha\text{-}]_D^{23}$ + 5,6° (Chloroform).

Beispiel 3

In 20 ml eisgekühltes absolutes Tetrahydrofuran werden 400 mg Lithium gepresst und anschliessend 7,8 ml 1-Brompentan zugetropft. Nach beendeter Reaktion werden 1,6 g 3,3-Ethylendioxy-1α-methyl-5α-androstan-17-on gelöst in 8 ml absolutem Tetrahydrofuran zugesetzt, und es wird 48 Stunden bei Raumtemperatur unter Argon nachgerührt. Es wird wie in Beispiel 2 aufgearbeitet. Nach Chromatographie an Silikagel werden 1,1 g 3,3-Ethylendioxy-1α-methyl-17α-n-pentyl-5α-androstan-17β-ol als Öl erhalten.

1,0 g 3,3-Ethylendioxy-1α-methyl-17α-n-pentyl-5α-androstan-17β-ol werden wie in Beispiel 2 zur Ketalspaltung eingesetzt und aufgearbeitet. Nach Chromatographie an Silikagel werden 720 mg 17β-Hydroxy-1α-methyl-17α-n-pentyl-5α-androstan-3-on als Öl erhalten. $[\alpha]_D^{23}$ + 4° (Chloroform).

Beispiel 4

In 30 ml eisgekühltes, absolutes Tetrahydrofuran werden 500 mg Lithium gepresst und anschliessend 11,3 ml 1-Bromhexan zugetropft. Nach beendeter Reaktion werden 2,0 g 3,3-Ethylendioxy-1α-methyl-5α-androstan-17-on in 10 ml absolutem Tetrahydrofuran zugetropft und 48 Stunden bei Raumtemperatur unter Argon gerührt. Es wird wie in Beispiel 2 aufgearbeitet und an Silikagel chromatographiert. Es werden 950 mg 3,3-Ethylendioxy-17α-n-hexyl-1α-methyl-5α-androstan-17β-ol als Öl erhalten.

850 mg 3,3-Ethylendioxy-17α-n-hexyl-1α-methyl-5α-androstan-17β-ol werden wie in Beispiel 2 unter Ketalspaltungsbedingungen umgesetzt und aufgearbeitet. Nach Chromatographie an Silikagel werden 630 mg 17α-n-Hexyl-17β-hydroxy-1α-methyl-5α-androstan-3-on als Öl erhalten. $[\alpha]_D^{23}$ + 4° (Chloroform).

Beispiel 5

5,0 g 17β-Hydroxy-1α-methyl-17α-n-propyl-5α-androstan-3-on werden in 5 ml absolutem Tetrahydrofuran mit 5,0 g Lithium-tri-tert.-butoxy-aluminiumhydrid 3 Stunden bei Raumtemperatur gerührt. Es wird mit Ether verdünnt, mit verdünnter Schwefelsäure und Wasser gewaschen, getrocknet und eingedampft. Nach Chromatographie an Silikagel und jeweils Umkristallisation aus Diisopropylether werden 860 mg 1α-Methyl-17α-n-propyl-5α-androstan-3β,17β-diol vom Schmelzpunkt 117–118°C und 3,3 g 3α-Isomeres vom Schmelzpunkt 143–144°C erhalten.

Beispiel 6

1,5 g 17β-Hydroxy-1α-methyl17α-n-propyl-5α-androstan-3-on werden in 6 ml Pyridin mit 3 ml Acetanhydrid nach Zugabe von 75 mg 4-Dimethylaminopyridin 16 Stunden bei Raumtemperatur stehengelassen. Nach Eiswasserfällung und Umkristallisation aus Hexan werden 1,3 g 17β-Acetoxy-1α-methyl-17α-n-propyl-5α-androstan-3-on vom Schmelzpunkt 128–129°C erhalten.

Beispiel 7

1,0 g 17β-Acetoxy-1α-methyl-17α-n-propyl-5α-androstan-3-on werden wie in Beispiel 5 mit Lithium-tri-tert.-butoxyaluminiumhydrid umgesetzt und aufgearbeitet. Nach Chromatographie an Silikagel werden 650 mg 17β-Acetoxy-1α-methyl-17α-n-propyl-5α-androstan-3β-ol als Öl erhalten.

Beispiel 8

250 mg 17β-Acetoxy-1α-methyl-17α-n-propyl-5α-androstan-3β-ol werden in 1 ml Pyridin mit 0,5 ml Buttersäureanhydrid 48 Stunden bei Raumtemperatur stehengelassen. Es wird mit Ether verdünnt, mehrmals mit Wasser gewaschen, getrocknet und eingedampft. Der Rückstand wird an Silikagel chromatographiert. Es werden 270 mg 17β-Acetoxy-3β-butyryloxy-1α-methyl-17α-n-propyl-5α-androstan als Öl erhalten.

Beispiel 9

400 mg 17β-Acetoxy-1α-methyl-17α-n-propyl-5α-androstan-3β-ol werden in 2,8 ml absolutem Dichlormethan und 1,8 ml Formaldehyddimethylacetal mit einer Mischung von 600 mg Kieselgur W 20 und 300 mg Phosphorpentoxid versetzt und 45 Minuten bei Raumtemperatur gerührt. Es wird von den unlöslichen Anteilen abgesaugt und mit Dichlormethan, das 3–5% Triethylamin enthält, nachgewaschen. Das nach dem Eindampfen erhaltene Rohprodukt wird an Silikagel chromatographiert. Es werden 280 mg 17β-Acetoxy-3β-

methoxy-methoxy-1α-methyl-17α-n-propyl-5α-androstan erhalten.

Beispiel 10

400 mg 17β-Hydroxy-1α-methyl-17α-n-propyl-5α-androstan-3-on werden in 1,6 ml Pyridin und 0,8 ml Önanthsäureanhydrid unter Zusatz von 40 mg 4-Dimethylaminopyridin 42 Stunden bei Raumtemperatur gerührt. Es wird mit Ether verdünnt, mit Wasser gewaschen, getrocknet und eingedampft. Der Rückstand wird an Silikagel chromatographiert. Es werden 370 mg 17β-Heptanoyloxy-1α-methyl-17α-n-propyl-5α-androstan-3-on als Öl erhalten.

Beispiel 11

7,0 g 3,3-Ethylendioxy-1α-methyl-5α-androstan-17-on werden in 70 ml Tetrahydrofuran mit 2,8 g Magnesiumspänen versetzt, dann werden langsam 14,35 ml Crotylbromid in 15 ml Tetrahydrofuran zugetropft und anschliessend wird 45 Minuten bei Raumtemperatur nachgerührt. Das überschüssige Reagenz wird bei Eiskühlung mit Ammoniumchloridlösung zersetzt, dann die Reaktionslösung mit Ether verdünnt, mit Wasser gewaschen, getrocknet und eingedampft. Der Rückstand wird an Silikagel chromatographiert, und es werden 1,95 g 3,3-Ethylendioxy-1α-methyl-17α-(1-methyl-2-propenyl)-5α-androstan-17β-ol als Rohprodukt erhalten.

1,92 g 3,3-Ethylendioxy-1α-methyl-17α-(1-methyl-2-propenyl)-5α-androstan-17β-ol werden in 19,2 ml Methanol mit 1,92 ml 8 Vol.%iger Schwefelsäure 15 Minuten bei Raumtemperatur gerührt. Es wird dann mit Ether verdünnt, mit Wasser neutral gewaschen, getrocknet und eingedampft. Der Rückstand wird an Silikagel chromatographiert, und es werden nach Umkristallisieren aus Diisopropylether 780 mg 17β-Hydroxy-1α-methyl-17α-(1-methyl-2-propenyl)-5α-androstan-3-on vom Schmelzpunkt 148,5–150°C erhalten.

770 mg 17β-Hydroxy-1α-methyl-17α-(1-methyl-2-propenyl)-5α-androstan-3-on werden in 5 ml Tetrahydrofuran und 15 ml Methanol mit 150 mg Palladium auf Kohle (10%ig) bis zur Aufnahme von einem Äquivalent Wasserstoff hydriert. Der Katalysator wird abfiltriert und das Filtrat im Vakuum eingedampft. Der Rückstand wird an Silikagel chromatographiert. Nach Umkristallisieren aus Diisopropylether werden 440 mg 17β-Hydroxy-1α-methyl-17α-(1-methyl-n-propyl)-5α-androstan-3-on vom Schmelzpunkt 172,5–173,5°C erhalten.

Beispiel 12

700 mg 1α-Methyl-17α-n-propyl-5α-androstan-3α,17β-diol (hergestellt nach Beispiel 5, Schmelzpunkt 143–144°C) werden in 2,8 ml Pyridin und 1,4 ml Essigsäureanhydrid 22 Stunden bei Raumtemperatur stehengelassen. Nach Eiswasserfällung wird das erhaltene Rohprodukt an Silikagel chromatographiert, und es werden 760 mg 3α-Acetoxy-1α-methyl-17α-n-propyl-5α-androstan-17β-ol als Öl erhalten.

Beispiel 13

1,5 g 1α-Methyl-17α-n-propyl-5α-androstan-3α,17β-diol werden in 6 ml Triethyylamin und 1,5 ml Essigsäureanhydrid mit 50 mg 4-Dimethylaminopyridin 6 Tage bei Raumtemperatur stehengelassen. Nach Eiswasserfällung wird das erhaltene Rohprodukt an Silikagel chromatographiert, und es werden 1,08 g 3α,17β-Diacetoxy-1α-methyl-17α-n-propyl-5α-androstan vom Schmelzpunkt 96–99°C erhalten.

Beispiel 14

1 g 3,3-Ethylendioxy-1α-methyl-17α-(2-propenyl)-5α-androstan-17β-ol (hergestellt gemäss Beispiel 1) werden in 10 ml Methanol mit 1 ml 8 Vol.%iger Schwefelsäure 15 Minuten bei Raumtemperatur gerührt und analog Beispiel 11 aufgearbeitet. Nach Umkristallisieren aus Diisopropylether erhält man 710 mg 17β-Hydroxy-1α-methyl-17α-(2-propenyl)-5α-androstan-3-on vom Schmelzpunkt 115–116°C.

**Patentansprüche für die Vertragsstaaten BE, CH, DE, FR, GB, IT, LI, LU, NL, SE**

1. 17α-Substituierte Steroide der allgemeinen Formel I

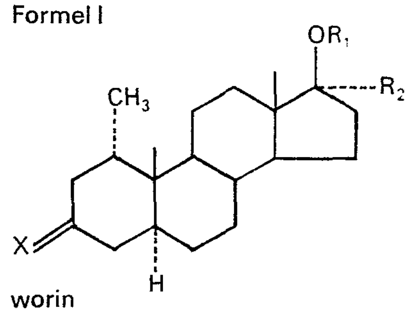

(I),

worin

$R_1$ ein Wasserstoffatom, eine Acyl-, eine gegebenenfalls durch ein Sauerstoffatom unterbrochene Alkylgruppe, eine Cyclopentyl- oder Tetrahydropyranylgruppe,

$R_2$ einen aliphatischen Kohlenwasserstoffrest mit 3-6 Kohlenstoffatomen,

X ein Sauerstoffatom oder die Gruppierung $H(OR_3)$, in der $R_3$ ein Wasserstoffatom, eine Acyl-, eine gegebenenfalls durch ein Sauerstoffatom unterbrochene Alkyl-, eine Cyclopentyl- oder Tetrahydropyranylgruppe

bedeuten.

2. 17β-Hydroxy-1α-methyl-17α-n-propyl-5α-androstan-3-on.

3. 17α-n-Butyl-17β-hydroxy-1α-methyl-5α-androstan-3-on.

4. 17β-Hydroxy-1α-methyl-17α-n-pentyl-5α-androstan-3-on.

5. 17α-n-Hexyl-17β-hydroxy-1α-methyl-5α-androstan-3-on.

6. 17β-Hydroxy-1α-methyl-17α-(2-propenyl)-5α-androstan-3-on.

7. 1α-Methyl-17α-n-propyl-5α-androstan-3β,17β-diol.

8. 17β-Acetoxy-1α-methyl-17α-n-propyl-5α-androstan-3-on.

9. 17β-Acetoxy-1α-methyl-17α-n-propyl-5α-androstan-3β-ol.

10. 17β-Acetoxy-3β-butyryloxy-1α-methyl-17α-n-propyl-5α-androstan.

11. 17β-Acetoxy-3β-methoxymethoxy-1α-methyl-17α-n-propyl-5α-androstan.

12. 17β-Heptanoyloxy-1α-methyl-17α-n-propyl-5α-androstan-3-on.

13. 17β-Hydroxy-1α-methyl-17α-(1-methyl-2-propenyl)-5α-androstan-3-on.

14. 17β-Hydroxy-1α-methyl-17α-(1-methyl-n-propyl)-5α-androstan-3-on.

15. 1α-Methyl-17α-n-propyl-5α-androstan-3α,17β-diol.

16. 3α-Acetoxy-1α-methyl-17α-n-propyl-5α-androstan-17β-ol.

17. 3α,17β-Diacetoxy-1α-methyl-17α-n-propyl-5α-androstan.

18. Pharmazeutische Präparate enthaltend eine oder mehrere Steroide gemäss Anspruch 1–17.

19. Verfahren zur Herstellung von 17α-substituierten Steroiden der allgemeinen Formel I gemäss Anspruch 1, dadurch gekennzeichnet, dass man ein 17-Ketosteroid der allgemeinen Formel II

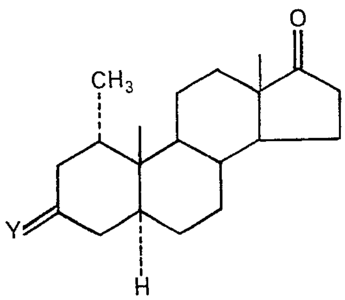

(II),

worin Y eine säurehydrolysierbare, geschützte 3-Oxogruppe bedeutet, mit einer den Rest $R_2$ abgebenden metallorganischen Verbindung umsetzt, gegebenenfalls eine Alkenylgruppe $R_2$ zur Alkylgruppe $R_2$ reduziert und die Schutzgruppe in 3-Stellung abspaltet und, je nach der letztlich gewünschten Bedeutung von $R_1$ und X im Endprodukt, gegebenenfalls die 17-Hydroxygruppe vor oder nach der Schutzgruppenabspaltung verestert oder verethert und/oder die 3-Ketogruppe reduziert und gegebenenfalls anschliessend freie Hydroxygruppen in 3-Stellung oder in 3- und 17-Stellung verestert oder verethert.

**Patentanspruch für den Vertragsstaat AT**

Verfahren zur Herstellung von 17α-substituierten Steroiden der allgemeinen Formel I gemäss Anspruch 1, dadurch gekennzeichnet, dass man ein 17-Ketosteroid der allgemeinen Formel II

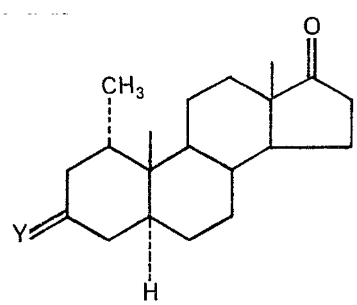

(II),

worin Y eine säurehydrolysierbare, geschützte 3-Oxogruppe bedeutet, mit einer den Rest $R_2$ abgebenden metallorganischen Verbindung umsetzt, gegebenenfalls eine Alkenylgruppe $R_2$ zur Alkylgruppe $R_2$ reduziert und die Schutzgruppe in 3-Stellung abspaltet und, je nach der letztlich gewünschten Bedeutung von $R_1$ und X im Endprodukt, gegebenenfalls die 17-Hydroxygruppe vor oder nach der Schutzgruppenabspaltung verestert oder verethert und/oder die 3-Ketogruppe reduziert und gegebenenfalls anschliessend freie Hydroxygruppen in 3-Stellung oder in 3- und 17-Stellung verestert oder verethert.

**Revendications pour les Etats contractants: BE, CH, DE, FR, GB, IT, LI, LU, NL, SE**

1. Les stéroïdes substitués à la position 17α de formule générale I ci-dessous

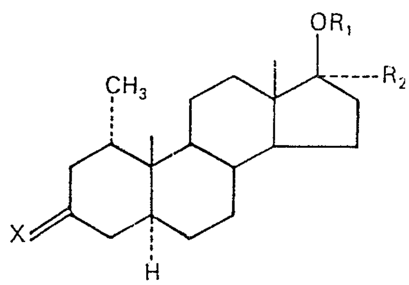

(I),

dans laquelle

$R_1$ représente un atome d'hydrogène, un groupe acyle, un alkyle éventuellement interrompu par un atome d'oxygène ou un groupe cyclopentyle ou tétrahydropyranyle,

$R_2$ représente un radical hydrocarboné aliphatique en $C_3$–$C_6$ et

X un atome d'oxygène ou un groupe $H(OR_3)$, $R_3$ désignant l'hydrogène, un groupe acyle, un alkyle éventuellement interrompu par un atome d'oxygène ou un groupe cyclopentyle ou tétrahydropyranyle.

2. 17β-Hydroxy-1α-méthyl-17α-n-propyl-5α-androstane-3-one.

3. 17α-n-Butyl-17β-hydroxy-1α-méthyl-5α-androstane-3-one.

4. 17β-Hydroxy-1α-méthyl-17α-n-pentyl-5α-androstane-3-one.

5. 17α-Hexyl-17β-hydroxy-1α-méthyl-5α-androstane-3-one.

6. 17β-Hydroxy-1α-méthyl-17α-(2-propényl)-5α-androstane-3-one.

7. 1α-Méthyl-17α-n-propyl-5α-androstane-3β,17β-diol.

8. 17β-Acétoxy-1α-méthyl-17α-n-propyl-5α-androstane-3-one.

9. 17β-Acétoxy-1α-méthyl-17α-n-propyl-5α-androstane-3β-ol.

10. 17β-Acétoxy-3β-butyryloxy-1α-méthyl-17α-n-propyl-5α-androstane.

11. 17β-Acétoxy-3β-méthoxyméthoxy-1α-méthyl-17α-n-propyl-5α-androstane.

12. 17β-Heptanoyloxy-1α-méthyl-17α-n-propyl-5α-androstane-3-one.

13. 17β-Hydroxy-1α-méthyl-17α-(1-méthyl-2-propényl)-5α-androstane-3-one.

14. 17β-Hydroxy-1α-méthyl-17α-(1-méthyl-n-propyl)-5α-androstane-3-one.

15. 1α-Méthyl-17α-n-propyl-5α-androstane-3α,17β-diol.

16. 3α-Acétoxy-1α-méthyl-17α-n-propyl-5α-androstane-17β-ol.

17. 3α,17β-Diacétoxy-1α-méthyl-17α-n-propyl-5α-androstane.

18. Préparations pharmaceutiques contenant comme matière active un ou plusieurs stéroïdes selon l'une quelconque des revendications 1 à 17.

19. Procédé de préparation des stéroïdes de formule générale I selon la revendication 1, procédé caractérisé en ce que l'on fait réagir un 17-cétostéroïde de formule générale II

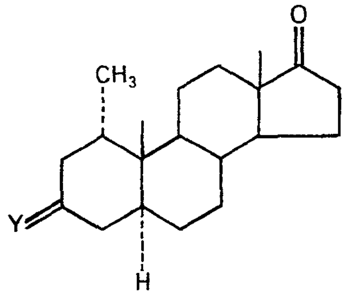

(II),

(Y désignant un groupe oxo en 3 protégé, hydrolysable en milieu acide) avec un composé organo-métallique apportant le radical $R_2$, éventuellement on réduit un groupe alcényle $R_2$ en un groupe alkyle, et on élimine le groupe protecteur à la position 3, puis, suivant ce que l'on veut obtenir dans le produit final en ce qui concerne $R_1$ et X, le cas échéant on estérifie ou on éthérifie le groupe 17-hydroxy avant ou après l'élimination du groupe protecteur, et/ou on réduit le groupe céto en 3, puis éventuellement on estérifie ou on éthérifie des hydroxyles libres à la position 3 ou aux positions 3 et 17.

**Revendications pour l'Etat contractant: AT**

Procédé de préparation de stéroïdes substitués à la position 17α, de formule générale I

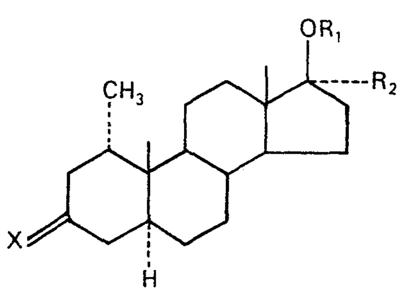

(I),

dans laquelle

$R_1$ représente un atome d'hydrogène, un groupe acyle, un alkyle éventuellement interrompu par un atome d'oxygène ou un groupe cyclopentyle ou tétrahydropyranyle,

$R_2$ représente un radical hydrocarboné aliphatique en $C_3$-$C_6$ et

X un atome d'oxygène ou un groupe $H(OR_3)$, $R_3$ désignant l'hydrogène, un groupe acyle, un alkyle éventuellement interrompu par un atome d'oxygène ou un groupe cyclopentyle ou tétrahydropyranyle,

procédé caractérisé en ce que l'on fait réagir un 17-cétostéroïde de formule générale II

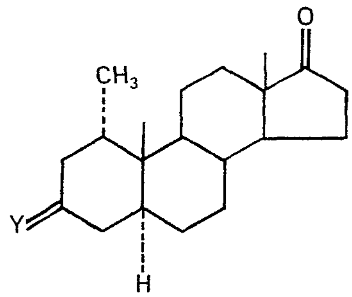

(II),

(Y désignant un groupe oxo en 3 protégé, hydrolysable en milieu acide) avec un composé organo métallique apportant le radical $R_2$, éventuellement on réduit un groupe alcényle $R_2$ en un groupe alkyle, et on élimine le groupe protecteur à la position 3, puis, suivant ce que l'on veut obtenir dans le produit final en ce qui concerne $R_1$ et X, le cas échéant on estérifie ou on éthérifie le groupe 17-hydroxy avant ou après l'élimination du groupe protecteur, et/ou on réduit le groupe céto en 3, puis éventuellement on estérifie ou on étherifie des hydroxyles libres à la position 3 ou aux positions 3 et 17.

**Claims for the Contracting States: BE, CH, LI, DE, FR, GB, IT, LU, NL, SE**

1. 17α-substituted steroids of the general formula I

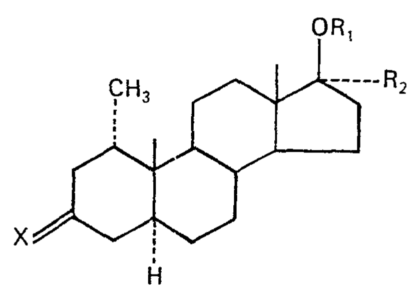

(I),

in which

$R_1$ represents a hydrogen atom, an acyl group, an alkyl group optionally interrupted by an oxygen atom, a cyclopentyl or tetrahydropyranyl group,

$R_2$ represents an aliphatic hydrocarbon radical having from 3 to 6 carbon atoms,

X represents an oxygen atom or the grouping $H(OR_3)$, in which $R_3$ represents a hydrogen atom, an acyl group, an alkyl group optionally interrupted by an oxygen atom, a cyclopentyl or tetrahydropyranyl group.

2. 17β-Hydroxy-1α-methyl-17α-n-propyl-5α-androstan-3-one.

3. 17α-n-Butyl-17β-hydroxy-1α-methyl-5α-androstan-3-one.
4. 17β-Hydroxy-1α-methyl-17α-n-pentyl-5α-androstan-3-one.
5. 17α-n-Hexyl-17β-hydroxy-1α-methyl-5α-androstan-3-one.
6. 17β-Hydroxy-1α-methyl-17α-(2-propenyl)-5α-androstan-3-one.
7. 1α-Methyl-17α-n-propyl-5α-androstan-3β,17β-diol.
8. 17β-Acetoxy-1α-methyl-17α-n-propyl-5α-androstan-3-one.
9. 17β-Acetoxy-1α-methyl-17α-n-propyl-5α-androstan-3β-ol.
10. 17β-Acetoxy-3β-butyryloxy-1α-methyl-17α-n-propyl-5α-androstan.
11. 17β-Acetoxy-3β-methoxymethoxy-1α-methyl-17α-n-propyl-5α-androstan.
12. 17β-Heptanoyloxy-1α-methyl-17α-n-propyl-5α-androstan-3-one.
13. 17β-Hydroxy-1α-(1-methyl-2-propenyl)-5α-androstan-3-one.
14. 17β-Hydroxy-1α-methyl-17α-(1-methyl-n-propyl)-5α-androstan-3-one.
15. 1α-Methyl-17α-n-propyl-5α-androstan-3α-17β-diol.
16. 3α-Acetoxy-1α-methyl-17α-n-propyl-5α-androstan17β-ol.
17. 3α,17β-Diacetoxy-1α-methyl-17α-n-propyl-5α-androstan.
18. Pharmaceutical preparations containing one or more steroids according to claims 1 to 17.
19. Process for the manufacture of 17α-substituted steroids of the general formula I according to claim 1, characterised in that a 17-keto steroid of the general formula II

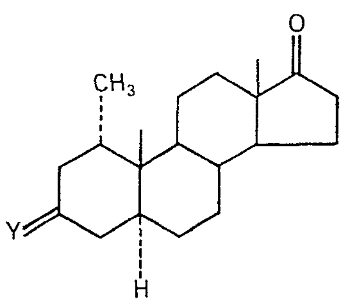

(II),

in which Y represents an acid-hydrolysable, protected 3-oxo group, is reacted with an organometallic compound giving off the radical $R_2$, optionally an alkenyl group $R_2$ is reduced to form the alkyl group $R_2$, and the protecting group in the 3-position is split off and, depending on the finally desired meaning of $R_1$ and X in the end product, optionally the 17-hydroxy group is esterified or etherified before or after splitting off the protecting groups and/or the 3-keto group is reduced and optionally subsequently free hydroxy groups in the 3-position or in the 3- and 17-positions are esterified or etherified.

**Claim for the Contracting State: AT**

Process for the manufacture of 17α-substituted steroids of the general formula I according to claim 1, characterised in that a 17-keto steroid of the general formula II

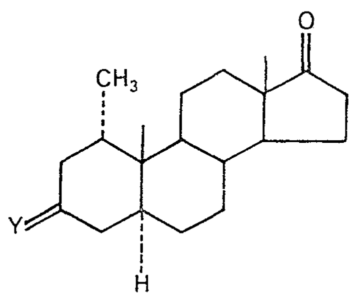

(II),

in which Y represents an acid-hydrolysable protected 3-oxo group is reacted with an organometallic compound giving off the radical $R_2$, optionally an alkenyl group $R_2$ is reduced to form an alkyl group $R_2$, and the protecting group in the 3-position is split off and, depending on the finally desired meaning of $R_1$ and X in the end product, optionally the 17-hydroxy group is esterified or etherified before or after splitting off the protecting groups and/or the 3-keto group is reduced and optionally subsequently free hydroxy groups in the 3-position or in the 3- and 17-positions are sterified or etherified.